# EUROPEAN PATENT SPECIFICATION

(11) **EP 3 389 676 B1**
(45) Date of publication and mention of the grant of the patent: **29.04.2026**
(21) Application number: 16809842.4
(22) Date of filing: 15.12.2016
(51) Int. Cl.: A61K 33/04, A61K 9/00, A61P 35/00, A61K 45/06

(54) **DOSAGE REGIMEN OF SODIUM SELENITE IN THE TREATMENT OF CANCER**
DOSIERUNGSPLAN FÜR NATRIUMSELENIT BEI DER BEHANDLUNG VON KREBS
RÉGIME POSOLOGIQUE DE SÉLÉNITE DE SODIUM DANS LE TRAITEMENT DU CANCER

(30) Priority: 16.12.2015 EP 15200465
(43) Date of publication of application: 24.10.2018
(73) Proprietor: Pharma Nord ApS, 6500 Vojens (DK)
(72) Inventor: MOESGAARD, Sven, 6051 Almind (DK); BJÖRNSTEDT, Mikael, 141 39 Huddinge (SE)
(74) Representative: COPA Copenhagen Patents
(86) International application number: PCT/EP2016/081234
(87) International publication number: WO 2017/102970

(56) References cited:
- WO-A2-2005/048925
- DE-A1- 19 825 746
- OLA BRODIN ET AL: "Pharmacokinetics and Toxicity of Sodium Selenite in the Treatment of Patients with Carcinoma in a Phase I Clinical Trial: The SECAR Study", NUTRIENTS, vol. 7, no. 6, 19 June 2015 (2015-06-19), pages 4978 - 4994, XP055257918, DOI: 10.3390/nu7064978
- BRODIN OLA ET AL: "A CLINICAL PHASE I STUDY ON ADMINISTRATION OF HIGH DOSE SODIUM SELENITE AS A TREATMENT OF CANCER THE SECAR STUDY", ANTICANCER RESEARCH - INTERNATIONAL JOURNAL OF CANCER RESEARCH AND TREATMENT; 9TH INTERNATIONAL CONFERENCE OF ANTICANCER RESEARCH; SITHONIA, GREECE; OCTOBER 06 -10, 2014, INTERNATIONAL INSTITUTE OF ANTICANCER RESEARCH, GR, vol. 34, no. 10, 1 October 2014 (2014-10-01), pages 5851 - 5852, XP002739152, ISSN: 0250-7005
- INAS A. ASFOUR ET AL: "High-Dose Sodium Selenite Can Induce Apoptosis of Lymphoma Cells in Adult Patients with Non-Hodgkin's Lymphoma", BIOLOGICAL TRACE ELEMENT RESEARCH, vol. 127, no. 3, 25 October 2008 (2008-10-25), pages 200 - 210, XP055185948, ISSN: 0163-4984, DOI: 10.1007/s12011-008-8240-6
- A. PAKDAMAN: "Symptomatic treatment of brain tumor patients with sodium selenite, oxygen, and other supportive measures", BIOLOGICAL TRACE ELEMENT RESEARCH, vol. 62, no. 1-2, 1 April 1998 (1998-04-01), pages 1 - 6, XP055185951, ISSN: 0163-4984, DOI: 10.1007/BF02820015
- AARON H ROSE ET AL: "GROWTH FACTORS, CYTOKINES, AND CELL CYCLE MOLECULES Increasing Dietary Selenium Elevates Reducing Capacity and ERK Activation Associated with Accelerated Progression of Select Mesothelioma Tumors From the Departments of Cell and Molecular Biology", AM J PATHOL, 1 January 2014 (2014-01-01), pages 1041 - 1049, XP055258035, Retrieved from the Internet <URL:http://ac.els-cdn.com/S0002944014000133/1-s2.0-S0002944014000133-main.pdf?_tid=bf0cf202-e9eb-11e5-b2ff-00000aab0f6c&acdnat=1457963623_36c4e8b0df9888ac1f99167544edc698>

## Description

### FIELD OF THE INVENTION

The present invention relates to a treatment regime wherein a human subject is treated with various doses of sodium selenite, and wherein the human subject may optionally further be treated with a cytostatic agent.

The present invention especially relates to the treatment of lung-lining cancer.

### BACKGROUND OF THE INVENTION

The ultimate goal of chemotherapy cure is rarely achieved in patients with advanced malignancies. The development of resistance to conventional drugs during therapy, used alone and in combination, remains a major obstacle to curative therapy.

The clinical challenge is to develop new drugs and treatment modalities that will affect cure rates significantly by e.g. reversing or preventing drug resistance, with minimal general and specific tumour cell toxicity.

Selenium compounds have been extensively evaluated as chemotherapy agents both alone and in combination with other drugs, but there have been few publications on the potential of these agents in modifying the toxicity and therapeutic efficacy of anti-cancer drugs against established tumours.

Cao et al. (Clinical Cancer Research, 2004) disclose that in mice bearing a human carcinoma, administration of high doses of selenium could serve as a modulator of the therapeutic efficacy of anti-cancer drugs. However, this reference also discloses that the toxicity of selenium is highly problematic. The kinetics of drug-induced toxicities including lethality were determined, and the therapeutic selectivity of chemotherapeutic agents in combination with a selenium dose below 0.2 mg/mouse/day was determined to be a safe and nontoxic dose and schedule.

However, the observed lethality of doses of above 0.2 mg/mice/day renders safe administration of similar or even close to similar doses to humans highly questionable.

The anti-neoplastic effects of selenium compounds are quite well established. Among different chemical forms of selenium, sodium selenite exhibits superior chemotherapeutic properties, as exemplified in many preclinical studies, both *in vivo* and *in vitro.* However, inorganic selenium, and in particular selenite, is believed to be much more toxic than organic selenium compounds.

Selenite is believed to induce multimodal cell death pathways depending on cell types, dose and exposure time *in vitro.* Several studies have reported a higher cytotoxicity of selenite towards cancer cells compared to the cytotoxicity towards their normal counterparts at sub-toxic concentration, thereby presenting a plausible therapeutic window.

It has further been shown that drug-resistant cancer cells are more sensitive to selenite than their drug-sensitive variants. In conjunction, in multiple experimental carcinogenesis and transplantable tumour models, a high dose of selenite has been found to be effective against both the tumour genesis and the progression of established carcinoma.

In spite of promising findings in preclinical investigations, the chemotherapeutic applications of selenite in cancer patients have been very limited. About a century ago, reports showed no incidence of toxic effects upon cumulative intravenous administration of 100 mg of the compound, and back in the 1930s, A.T. Todd also demonstrated remarkable effects of colloidal lead selenide in patients with inoperable carcinoma. Nevertheless, no systematic follow-up clinical studies have been carried out to evaluate the chemotherapeutic potential of redox-active inorganic selenium compounds.

The absence of pertinent pharmacokinetics data and the high toxicity of selenite in humans have limited its potential chemotherapeutic application. This necessitates comprehensive dose-response kinetics evaluations of selenite in humans.

Brodin et al., 2015 Nutrients 7, 4978-4994, show pharmacokinetics and toxicity of sodium selenite in the treatment of patients with carcinoma in a phase I clinical trial: The SECAR Study, which determine the MTD of iv administrated sodium selenite in terminal cancer patients.

Brodin et al. reported in Anticancer Research 2014 (vol. 34, no. 10, pages 5851-5852) on the SECAR clinical phase I study showing administration of high dose sodium selenite as a treatment of cancer. This study used intermittent daily infusions (20-40 minutes) with dose escalation protocols to determine the maximum tolerated dose of approximately 10.2 mg/m². While recognizing the need for 'prolonged selenite infusion' to achieve better therapeutic effects, the study did not disclose any specific continuous infusion regimen or the two-phase dosing approach of the present invention.

Furthermore, evidence suggests that selenite is an agent that exhibits carcinogenic/mutagenic properties. Selenite in tracer doses is necessary for cells both benignant and malignant cells to survive the effect of toxic substances such as free radicals. Since malignant cells as a rule have a higher metabolism and by that produce more free radicals than benignant ones, they need more selenite and have developed mechanisms to satisfy that need. However, a supply above a certain level is toxic, but below this level, a generous supply might even stimulate the tumour to grow faster.

Thus, great care must be taken to avoid further stimulation of the growth of tumours when administering selenite to a patient in late-stage treatment.

Thus, though the use of inorganic selenium compounds in the treatment of cancer conceptually is known in the field of cancer therapy, there is an ongoing need to identify dosage regimen that balance the potential curative effects of the inorganic selenium compounds and the extreme toxicity.

Thus, there is a need to develop new dosage regimen that e.g. reverse drug resistance, while enhancing therapeutic selectivity and cure rates.

### SUMMARY OF THE INVENTION

The present invention relates to a dosage regime of the safe and non-toxic, but therapeutically effective administration of sodium selenite to cancer patients.

The present inventors discovered that sodium selenite should be given as a prolonged continuous supply of sodium selenite during for example 48 to 72 hours, e.g. 3 - 30 mg/m² during 48 hours or e.g. 4 - 40 mg/m² during 72 hours. This treatment should be given with a short loading phase of less than 5 hours followed by a long-term phase for at least 24 hours, preferably 48 or 72 hours.

Accordingly, the present invention discloses sodium selenite for use in the treatment of cancer, wherein the sodium selenite is administered in a dosage regime comprising
a) a short loading phase comprising infusion of a dose of sodium selenite of 0.125-3 mg/m² per hour for less than 5 hours wherein a total dose of 3-9 mg/m² is administered during a period of 3 hours followed by
b) a long-term phase comprising infusion of a dose of sodium selenite of 0.333-1.1 mg/m² per hour for at least 24 hours,
wherein the infusion dose of sodium selenite per hour in the short loading phase is at least 2 times higher than the infusion dose of sodium selenite per hour in the long-term phase.

When administering sodium selenite in the specific dosage regime, the sodium selenite also increases the anti-tumour activity of anti-cancer agents.

Accordingly, the present invention further discloses sodium selenite for use in the treatment of cancer, wherein the sodium selenite is administered in a dosage regime comprising
a) a short loading phase comprising infusion of a dose of sodium selenite of 0.125-3 mg/m² per hour for less than 5 hours wherein a total dose of 3-9 mg/m² is administered during a period of 3 hours followed by
b) a long-term phase comprising infusion of a dose of sodium selenite of 0.333-1.1 mg/m² per hour for at least 24 hours,
wherein the infusion dose of the sodium selenite per hour in the short loading phase is at least 2 times higher than the infusion dose of the sodium selenite per hour in the long-term phase, wherein the human subject is further treated with a cytostatic agent.

It should be understood that any feature and/or aspect discussed herein in connection with the compounds according to the invention also applies by analogy to methods of treatment.

Accordingly, the present disclosure also relates to methods of treatment comprising administering selenite in a dosage regime of the present invention optionally together with an anti-tumour agent to an individual suffering from cancer.

The sodium selenite dosage regimen of the present invention may be administered before, during or after administration of the anti-cancer agent.

In a preferred embodiment, the sodium selenite dosage regimen is initiated prior to the chemotherapy to prime the tumour.

### BRIEF DESCRIPTION OF THE FIGURES

Embodiments of the invention will be described in more detail in the following with regard to the accompanying figures. The figures show one way of implementing the present invention and are not to be construed as being limiting to other possible embodiments falling within the scope of the attached claim set.

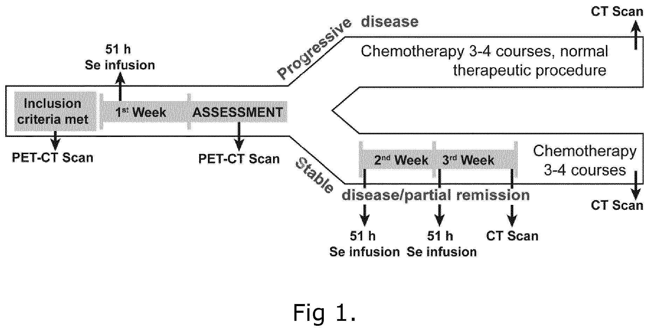

Fig. 1 is a schematic diagram depicting a treatment design constituting one to three dosage (51 hours) regimens according to the present invention.

### DETAILED DESCRIPTION OF THE INVENTION

The present invention discloses a clinical dosage regime of infusion of sodium selenite in cancer patients.

### Two step dosage regimes

The present inventors define the pharmacokinetics, which provide essential information for the use of sodium selenite in the treatment of cancer patients.

The present invention relates to the effect of dosage regimes of sodium selenite as an interesting option for the treatment of tumours and an efficient complement for cytostatic regimes.

The present disclosure relates to a method of treatment of cancer comprising a prolonged infusion of e.g. 2-3 days (48-72 hours) of a sufficiently high and continuous dose of selenite at a level of e.g. 3 mg/m² up to 30 mg/m² during for example 48 hours of iv treatment or 4-40 mg/m² during 72 hours of iv treatment.

Basically, this one main objective of the present invention (i.e. to allow patients to be pretreated with selenite such that the selenite exposure is prolonged, e.g. preferably 2 or 3 days, and such that the dose is sufficiently high and continuous, through intravenous supply of sodium-selenite, to reach a constant plasma level of at least 4.5 µmol for at least 2 days.

This objective is preferably obtained according to the invention by administering sodium selenite via infusions, in a dosing regimen comprising
a) a short loading phase comprising infusion of a dose of sodium selenite of 0.125-2 mg/m² per hour for less than 5 hours followed by
b) a long-term phase comprising infusion of a dose of sodium selenite of 0.333-0.7 mg/m² per hour for at least 48 hours

The dosing regimen may also preferably comprise a third observation phase in which, during and a few hours after such selenite treatment, the patient is continuously observed because of the risk of serious toxicity in the brain or the heart as well as an acute risk of tumour necrosis that might make the tumour swell which might give serious symptoms in some patients.

The invention relates to sodium selenite for use in the treatment of cancer, wherein the selenite is administered in a dosage regime comprising:
a) a short loading phase comprising infusion of a dose of sodium selenite of 0.125-3 mg/m² per hour for less than 5 hours wherein a total dose of 3-9 mg/m² is administered during a period of 3 hours followed by
b) a long-term phase comprising infusion of a dose of sodium selenite of 0.333-1.1 mg/m² per hour for at least 24 hours,
wherein the infusion dose of sodium selenite per hour in the short loading phase is at least 2 times higher than the infusion dose of sodium selenite per hour in the long-term phase.

As the skilled addressee would be aware, there is significant risk of toxic reactions using high doses of inorganic selenium in patients. Thus, the medical staff administering the inorganic selenium compound in the dosage regimen of the present invention should provide for a warning system alerting if acute toxicity occurs in the brain and heart, but also if acute tumour necrosis occurs.

The present invention further envisages that continuous observation of the patient during and immediately after a treatment is required.

Thus, the present invention also relates to sodium selenite for use in a method of treatment of cancer, wherein sodium selenite is administered in a dosage regime having a sufficiently high and continuous intravenous supply of sodium-selenite to reach a plasma level of for example at least 4.5 micro mol, wherein said method has
a) a short loading phase comprising infusion of a dose of sodium selenite of 0.125-3 mg/m² per hour for less than 5 hours wherein a total dose of 3-9 mg/m² is administered during a period of 3 hours followed by
b) a long-term phase comprising infusion of a dose of sodium selenite of 0.333-1.1 mg/m² per hour for at least 24 hours
c) and optionally during, and a few hours after, such a treatment continuously observing the patient
wherein the infusion dose of sodium selenite per hour in the short loading phase is at least 2 times higher than the infusion dose of sodium selenite per hour in the long-term phase.

The continuous observation of the patient is recommendable due to the risk of serious toxicity in the brain or heart as well as the acute risk of tumour necrosis that might make the tumour swell, which might give serious symptoms in some patients.

The present inventors completed clinical studies wherein all patients had tumours resistant to established treatment and progressing.

In one of these patients, being administered a daily dose of 0.9 mg daily during a period of 10 days, the tumour progressed with a significant increase of bone metastases and pain, which shows that an accelerated tumour growth during these weeks cannot be excluded. Since the tumours accelerate without obvious reasons, it cannot be excluded that low to moderate doses of selenite might stimulate tumour growth to an extent that is clinically important.

The overall strategy of the present invention is to show that a continuous treatment and/or repeated infusions of sodium selenite optionally prior to chemotherapy is advantageous.

One object of preferred embodiments and a technical differentiating factor of this invention is the splitting of the dosage regime into two phases; one being a "short loading phase" and the second being a subsequent continuous treatment or "long term phase", wherein the infusion dose of the inorganic selenium in the short loading phase is higher than the long-term phase.

The technical rational of the split is the balance between the toxicity of the selenium vs. the risk that lower dosages of selenium might actually have a tumour-promoting effect or no effect on the tumour.

The present invention works over a longer period of time than standard treatments of inorganic selenium [the long-term phase], but at doses that still keep the dosages higher than dosages of selenium which might actually have a tumour-promoting effect or no effect on the tumour. These risks are prevented by including the short loading phase, wherein the desired plasma concentration of the inorganic selenium is achieved quickly, and then maintained during the long-term phase. This "two step treatment" provides a beneficial effect.

Thus, the relatively higher "short loading phase dose" compared to the lower "long-term phase" described herein results in a specified amount of selenium reaching the blood stream of the patient quickly, thereby priming the patient to be receptive to the continuous flow of inorganic selenium, thereby enabling each patient to cope with higher doses.

It is a presently preferred embodiment that the infusion dose of selenite per hour in the short loading phase is at least 2 times higher than the infusion dose of the selenite per hour in the long term phase, such as but not limited to at least 2.25 times higher, at least 2.5 times higher, at least 2.75 times higher, at least 3 times higher, at least 3.25 times higher, at least 3.5 times higher, at least 3.75 times higher, at least 4 times higher, at least 4.25 times higher, at least 4.5 times higher, at least 4.75 times higher, at least 5 times higher, or more.

### Combination dosage regime

By combining traditional chemotherapy with the administration of inorganic selenium compounds in the dosage regime described in the present application, the anti-tumour toxicity of the chemotherapeutic agent can be increased.

Thus, another aspect of the present invention relates to sodium selenite for use in the treatment of cancer, wherein the selenite is administered in a dosage regime comprising
a) a short loading phase comprising infusion of a dose of sodium selenite of 0.125-3 mg/m² per hour for less than 5 hours wherein a total dose of 3-9 mg/m² is administered during a period of 3 hours followed by
b) a long-term phase comprising infusion of a dose of sodium selenite of 0.333-1.1 mg/m² per hour for at least 24 hours,
wherein the infusion dose of sodium selenite per hour in the short loading phase is at least 2 times higher than the infusion dose of sodium selenite per hour in the long-term phase, wherein the human subject is further treated with a cytostatic agent.

The present invention also relates to a combination dosage regimen as described herein comprising a cytostatic agent and sodium selenite together with a pharmaceutically acceptable carrier for use in the treatment of cancer in a human.

The sodium selenite may be administered before, during or after administration of the anti-cancer agent.

Considering the advanced disease status and acquired resistance to the conventional chemotherapeutic treatments prior to the enrolment in some patients, objective responses and stable diseases in any patients are quite encouraging.

### Synergistic effect

Selenite by itself is efficient in targeting certain tumours. However, a more pronounced effect of selenium administration may be seen after subsequent chemotherapy.

In one embodiment, the present invention relates to the synergistic effect of the 2-step dosage infusion of sodium selenite in combination with a cytostatic agent.

Interestingly, after receiving selenite treatment, many of the patients responded again to their first line of chemotherapy, following which patients were considered to have symptomatic benefits of the treatment. These *in vivo* effects corroborated well with the findings of the *in vitro* study that demonstrated increased sensitivity towards identical chemotherapeutic drugs to primary tumour cells derived from the patient.

It should be noted that selenite and dosage regimes according to the present invention - when administered intravenously to a human - were found not to be toxic, but rather proved to be effective modulators of toxicity and efficiency of the anti-cancer drugs.

Thus, it was demonstrated that sodium selenite potentiates the cure rate in humans suffering from both drug-sensitive and relatively resistant tumours. The *in vivo* effects were observed using non-toxic doses of the selenite.

While not intending to be bound by any particular theory, it is considered that potentiation of the efficacy of anti-cancer drugs is associated with increased anti-tumour activity and decreased toxicity.

### Plasma concentration

The pharmacokinetic data disclose a linear increase in the plasma selenite concentration with respect to the total dose. However, the plasma selenite concentration returned to the baseline levels at the end of the treatment, indicating no apparent adverse effects of high doses of repeated selenite administration on physiological selenium homeostasis.

In one embodiment, the infusion of selenite in the dosage regimen of the present invention provides a resulting plasma concentration of the selenite between 8-30 microM, such as but not limited to a plasma concentration in the range of 8 microM, 9 microM, 10 microM, 11 microM, 12 microM, 13 microM, 14 microM, 15 microM, 16 microM, 17 microM, 18 microM, 19 microM, 20 microM, 21 microM, 22 microM, 23 microM, 24 microM, 25 microM, 26 microM, 27 microM, 28 microM, 29 microM, and/or 30 microM.

In a preferred embodiment, the plasma concentration remains relatively constant during the long-term phase.

With respect to selenite toxicity, our findings were similar to what has been described earlier, i.e. toxicity mainly observed as nausea, vomiting and fatigue, side effects that could be dose-limiting in spite of antiemetic treatment. The most significant adverse effects were heart toxicity with signs of ischemia, confusion and unconsciousness especially at high dose levels such as beyond MTD.

The dose-limiting toxicity (DLT) appeared some hours after selenite infusion and was of a short duration, i.e. not more than up to 6-8 hours, without persisting toxicity or sequelae.

The pharmacokinetics data demonstrated a dose-dependent increase in the plasma selenite concentration. However, the highest peak concentration reached a plateau at dose level ≥ 10.2 mg/m².

Notably, a variable infusion time at these dose levels provided a window for plasma clearance and thereby precluded any direct comparison. In one patient (dose level 12.3 mg/m²), the renal clearance was 46% of the total selenium dose administered, suggesting an important role of the kidney in the excretion of selenium from selenite.

It is obviously desirable to keep MTD individual for each patient.

### The short loading phase

As described above, its desirable that the short loading phase infusion results in selenium reaching the blood stream of the patient quickly, which can be achieved by infusion of a dose of selenite of 0.125-3 mg/m² per hour for less than 5 hours, such as less than 4 hours, less than 3 hours, less than 2 hours or less than 1 hour.

In a presently preferred embodiment, the short loading phase consists of an infusion solution administered in a total dose of 3-9 mg/m² during a period of 3 hours.

In one embodiment, the short loading phase infusion dose of the sodium selenite is selected from the group consisting of an infusion solution administered in a dose of 0.1333 mg/m² per hour, 0.2667 mg/m² per hour, 0.4 mg/m² per hour, 0.5 mg/m² per hour, 0.7667 mg/m² per hour, 1.0 mg/m² per hour, 1.2667 mg/m² per hour, 1.5 mg/m² per hour, 1.7667 mg/m² per hour, 2.0 mg/m² per hour, 2.25 mg/m² per hour, 2.5 mg/m² per hour, 2.75 mg/m² per hour and 3.0 mg/m² per hour.

The range of the dose of the sodium selenite in the loading phase of 0.125-3 mg/m² per hour may for example be between 0.125-0.25 mg/m² per hour, 0.125-0.4 mg/m² per hour, 0.125-0.5 mg/m² per hour, 0.125-0.767 mg/m² per hour, 0.125-1 mg/m² per hour, 0.125-1.125 mg/m² per hour, 0.125-1.5 mg/m² per hour, 0.125-1.767 mg/m² per hour, 0.125-2 mg/m² per hour, 0.125-2 mg/m² per hour, 0.125-2,25 mg/m² per hour, 0.125-2,5 mg/m² per hour, 0.125-2,75 mg/m² per hour, 0.125-3 mg/m² per hour, 0.25-0.4 mg/m² per hour, 0.25-0.5 mg/m² per hour, 0.25-0.767 mg/m² per hour, 0.25-1 mg/m² per hour, 0.25-1.125 mg/m² per hour, 0.25-1.5 mg/m² per hour, 0.25-1.767 mg/m² per hour, 0.25-2 mg/m² per hour, 0.25-2,25 mg/m² per hour, 0.25-2,5 mg/m² per hour, 0.25-2,75 mg/m² per hour, 0.25-3 mg/m² per hour, 0.5-0.767 mg/m² per hour, 0.5-1 mg/m² per hour, 0.5-1.125 mg/m² per hour, 0.5-1.5 mg/m² per hour, 0.5-1.767 mg/m² per hour, 0.5-2 mg/m² per hour, 0.5-2,25 mg/m² per hour, 0.5-2,5 mg/m² per hour, 0.5-2,75 mg/m² per hour, 0.5-3 mg/m² per hour, 0.767-1 mg/m² per hour, 0.767-1.125 mg/m² per hour, 0.767-1.5 mg/m² per hour, 0.767-1.767 mg/m² per hour, 0.767-2 mg/m² per hour, 0.767-2,25 mg/m² per hour, 0.767-2,5 mg/m² per hour, 0.767-2,75 mg/m² per hour, 0.767-3 mg/m² per hour, 1-1.125 mg/m² per hour, 1-1.5 mg/m² per hour, 1-1.767 mg/m² per hour, 1-2 mg/m² per hour, 1.125-1.5 mg/m² per hour, 1.125-1.767 mg/m² per hour, 1.125-2 mg/m² per hour, 1.125-2,25 mg/m² per hour, 1.125-2,5 mg/m² per hour, 1.125-2,75 mg/m² per hour, 1.125-3 mg/m² per hour, 1.5-1.767 mg/m² per hour, 1.5-2 mg/m² per hour, 1.767-2 mg/m² per hour, 1.767-2,25 mg/m² per hour, 1.767-2,5 mg/m² per hour, 1.767-2,75 mg/m² per hour, 1.767-3 mg/m² per hour, 2-2,25 mg/m² per hour, 2-2,5 mg/m² per hour, 2-2,75 mg/m² per hour, 2-3 mg/m² per hour, 2,25-2,5 mg/m² per hour, 2,25-2,75 mg/m² per hour, 2,25-3 mg/m² per hour, 2,5-2,75 mg/m² per hour, 2,5-3 mg/m² per hour, 2,75-3 mg/m² per hour, or any interval selected by the physician in the clinic.

In a presently preferred embodiment, the short loading phase is 3 hours.

In a presently preferred embodiment, the dose of sodium selenite in the short loading phase over 3 hours is selected from the group consisting of 0.125 mg/m² per hour, 0.25 mg/m² per hour, 0.5 mg/m² per hour, 0.767 mg/m² per hour, 1 mg/m² per hour, 1.125 mg/m² per hour, 1.5 mg/m² per hour, 1.767 mg/m² per hour 2 mg/m² per hour, 2.25 mg/m² per hour, 2.5 mg/m² per hour, 2.75 mg/m² per hour, and 3 mg/m² per hour.

### The long term phase

In one embodiment, the long term phase infusion dose of sodium selenite is selected from the group consisting of an infusion solution administered in a dose of 0.3333 mg/m² per hour, 0.4167 mg/m² per hour, 0.5 mg/m² per hour, 0.5833 mg/m² per hour, 0.6667 mg/m² per hour, 0.75 mg/m² per hour, 0.85 mg/m² per hour, 0.95 mg/m² per hour, 1 mg mg/m² per hour, and 1.1 mg/m² per hour.

As described above, it is desirable that the long term phase infusion results in the selenium reaching the blood stream of the patient continuously over a period of at least 24 hours, such as at least 25 hours, at least 26 hours, at least 27 hours, at least 28 hours, at least 29, hours, at least 30 hours, at least 31 hours, at least 32 hours, at least 33 hours, at least 34 hours, at least 35 hours, at least 36 hours, at least 37 hours, at least at least 38 hours, at least 39 hours, at least 40 hours, at least 41 hours, at least 42 hours, at least 43 hours, at least 44 hours, at least 45 hours, at least 46 hours, at least 47 hours, at least 48 hours, at least 49 hours, at least 50 hours, at least 51 hours, at least 52 hours, at least 53 hours, at least 54 hours, at least 55 hours, at least 56 hours, at least 57 hours, at least 58 hours, at least 59 hours, at least 60 hours, at least 61 hours, at least 62 hours, at least 63 hours, at least 64 hours, at least 65 hours, at least 66 hours, at least 67 hours, at least 68 hours, at least 69 hours, at least 70 hours, at least 71 hours, at least 72 hours, at least 73 hours, at least 74 hours, at least 75 hours, at least 76 hours, at least 77 hours, at least 78 hours, at least 79 hours, at least 80 hours, at least 81 hours, at least 82 hours, at least 83 hours, at least 84 hours, at least 85 hours, at least 86 hours, at least 87 hours, at least 88 hours, at least 89 hours, at least 90 hours, at least 91 hours, at least 92 hours, at least 93 hours, at least 94 hours, at least 95 hours, at least 96 hours, at least 97 hours, at least 98 hours, at least 99 hours, at least 100 hours, at least 101 hours, at least 102 hours, at least 103 hours, at least 104 hours, at least 105 hours, at least 106 hours, at least 107 hours, at least 107 hours, at least 108 hours, at least 109 hours, at least 110 hours, at least 111 hours, at least 112 hours, at least 113 hours, at least 114 hours, at least 115 hours, at least 116 hours, at least 117 hours, at least 118 hours, at least 119 hours, at least 120 hours or more.

In a presently preferred embodiment, the long-term phase infusion phase is 48 hours.

In another presently preferred embodiment, the long-term phase infusion phase is 72 hours.

In one embodiment, the long-term infusion phase consists of an infusion solution administered in a total dose of 2-53 mg/m² during a period of 48 hours.

In another embodiment, the long-term infusion phase consists of an infusion solution administered in a total dose of 3-80 mg/m² during a period of 72 hours.

The range of the dose of sodium selenite in the long term phase is 0.333-0-417 mg/m² per hour, 0.333-0.5 mg/m² per hour, 0.333-0.583 mg/m² per hour, 0.333-0.667 mg/m² per hour, 0.333-0.75 mg/m² per hour, 0.333-0.85 mg/m² per hour, 0.333-0.95 mg/m² per hour, 0.333-1 mg/m² per hour, 0.333-1.1 mg/m² per hour, 0.417-0.5 mg/m² per hour, 0.417-0.583 mg/m² per hour, 0.417-0.667 mg/m² per hour, 0.417-0.75 mg/m² per hour, 0.417-0.84 mg/m² per hour, 0.417-0.95 mg/m² per hour, 0.417-1 mg/m² per hour, 0.417-1.1 mg/m² per hour, 0.5-0.585 mg/m² per hour, 0.5-0.667 mg/m² per hour, 0.5-0.667 mg/m² per hour, 0.5-0.667 mg/m² per hour, 0.5-0.75 mg/m² per hour, 0.5-0.85 mg/m² per hour, 0.5-0.95 mg/m² per hour, 0.5-1 mg/m² per hour, 0.5-1,1 mg/m² per hour, 0.583-0.667 mg/m² per hour, 0.583-0.75 mg/m² per hour, 0.583-0.75 mg/m² per hour, 0.583-0.85 mg/m² per hour, 0.583-0.95 mg/m² per hour, 0.583-1 mg/m² per hour, 0.583-1.1 mg/m² per hour, 0.667-0.75 mg/m² per hour, 0.667-0.85 mg/m² per hour, 0.667-0.95 mg/m² per hour, 0.667-1 mg/m² per hour, 0.667-1.1 mg/m² per hour, 0.75-0.85 mg/m² per hour, 0.75-0.95 mg/m² per hour, 0.75-1 mg/m² per hour, 0.75-1.1 mg/m² per hour, 0.85-0.95 mg/m² per hour, 0.85-1 mg/m² per hour, 0.85-1.1 mg/m² per hour, 0.95-1 mg/m² per hour, 0.95-1.1 mg/m² per hour, and 1-1.1 mg/m² per hour.

The table below shows some of the preferred regimes of the invention.

**Table 1: Two days of continuous treatment**

| **Loading dose** - **during 3 h (mg/m²)** | **Continuous dose during 24 h/ day 1 (mg/m²)** | **Continuous dose during 24 h/ day 2 (mg/m²)** | **Total dose in 51 hours (mg/m²)** | **Toxicity registered from similar dose, during similar time but as intermittent infusion** |
|---|---|---|---|---|
| 0.4 | 1 | 1 | 2.4 | 3.0 mg/m², cohort 2, no certain toxicity, |
| 0.8 | 2 | 2 | 4.8 | 4.5 mg/m², cohort 3, very mild toxicity (2.5 µM sodium selenite) |
| 1.2 | 3 | 3 | 7.2 | 6.0 mg/m², cohort 4, mild toxicity |
| 1.5 Cohort 12 | 4 | 4 | 9.5 (Start-dose) | 9.0 mg/m2, cohort 5, gr 1 toxicity. (5.0 µM sodium selenite) |
| 2.3 | 6 | 6 | 14.3 | 13.5 mg/m² cohort 6 |
| 3 | 8 | 8 | 19 | 20.4 mg/m², cohort 7, grade 2 toxicity (10.0 µM sodium selenite) |
| 3.8 | 10 | 10 | 23.8 | |
| 4.5 | 12 | 12 | 28.5 | 30.6 mg/m2, cohort 8, MTD for repeated infusions, (15.0 µM) |
| 5.3 | 14 | 12 | 33.3 | - |
| 6 | 16 | 16 | 38 | 38.4 mg/m², cohort 10, gr 3 & gr 4 toxicity |

### Evaluation step

Patients underwent CT and/or PET-CT examination at various stages during the treatment, such as but not limited to one week before the start, three days after the completion of the selenite treatment, and after the final chemotherapy treatment.

Routine blood parameters were analysed before, during and after the treatment.

The ECG was examined before the start and after both the selenite and chemotherapy treatments. There are data indicating an interaction between selenite and actin, one of the main components of skeletal and heart muscles. Extreme fatigue and heart toxicity indicating an impaired heart function has been registered during treatment with selenite at a high dose. Even if the toxicity seems to be completely reversible, even a short impairment of heart function might be lethal, and continuous registration of the heart function during prolonged selenite infusion seems recommendable.

Thus, in one embodiment, the administration pattern(s) of the present invention is followed by an evaluation step; preferably, the evaluation step consists of a PET-CT scanning.

In some embodiments, the treatment with inorganic selenium is stopped if the evaluation step discloses disease progression.

### Repeated infusions

The present invention also relates to repeated infusions schemes prior to chemotherapy.

Fig 1 shows a diagram over one presently preferred way of administrating the infusion 3 times over 3 weeks prior to chemotherapy. This is of course individually from patient to patient and can be based on the outcome of the evaluation steps by a skilled addressee.

It may be advantageous to repeat the present dosage regimen a few times, especially prior to the combination dosage regimen with a chemotherapeutic agent. Thus. in one embodiment the treatment regimen is repeated one, two, or three times or more.

The administration of sodium selenite dosage regimen according to the present invention can be initiated before the start of chemotherapy, during chemotherapy or after cessation of chemotherapy.

If initiated before the start of chemotherapy, the administration of sodium selenite can be continued during the chemotherapy and after cessation of chemotherapy.

Similarly, if initiated during chemotherapy, the administration of sodium selenite can continue after cessation of chemotherapy.

In one embodiment, the present invention relates to the administration of sodium selenite before, during or after administration of the anti-cancer agent.

In a presently preferred embodiment, the human subject is treated with sodium selenite for a time-period of at least 2 days before treatment with the cytostatic agent.

In one embodiment, the present invention relates to the administration of the specified amount of sodium selenite over a total for a period of between 5 and 20 days but can easily be extended up two months.

In some embodiments, the specified amount of sodium selenite is administered in one or more dosages, administered once, twice, three, or four times a day.

In some embodiments, the specified amount of sodium selenite is administered once daily as a continuous administration.

In some embodiments, the specified amount of sodium selenite is administered in two dosages (twice) daily.

In some embodiments, the sodium selenite is administered in different doses, wherein at least one of the administered doses differs from another administered dose.

Thus, the present invention relates the use of selenite.

In an embodiment, the selenite is sodium selenite.

### Administration of sodium selenite

The required dose is administered by infusion (such as intravenous or intra-tumour) administration.

In one embodiment, the present invention relates to *intra-tumoural* delivery by injection or by a nano-needle array.

In a presently preferred embodiment, the sodium selenite of the present invention is administered intravenously as an infusion solution.

### The body surface area

Many cancer drugs are toxic, and the range between maximum benefit and severe side effects is narrow. Hence, doses have to be adjusted for the size of the patient's body.

The body surface area (BSA) is the measured or calculated surface area of a human body. For many clinical purposes, BSA is a better indicator of metabolic mass than body weight because it is less affected by abnormal adipose mass. The dosages in the present invention are indicated in BSA. Various calculations have been published to arrive at the BSA without direct measurement. The most widely used is the Du Bois formula.

The sodium selenite for use in a dosage regime of the present invention can be used in a method for treating a human patient suffering from cancer, said method comprising administering therapeutically effective amounts of said sodium selenite in a dosage regime to such a patient for a period of time sufficient to trigger at least a partial tumour response.

### Maximum-tolerated dose (MTD)

In a previous study, the present inventors carried out the first in-human phase-I trial to determine the MTD of intravenously administered sodium selenite in patients with advanced cancer diseases. Sodium selenite was well tolerated in these patients at doses up to 10.2 mg/m², therefore defined as MTD.

At this dose and below, there were limited incidences of adverse effects. Routine blood sample analyses indicated limited side effects, which, if observed at all, were transient in nature. Notably, no bone marrow toxicity was evident.

Thus, in one embodiment, the dosage regime of the present invention aims at administering sodium selenite intravenously in a dose below the MTD.

In the context of the present invention, the dose limiting toxicity (DTL) is less than 26.4 mg/m² per day with regard to the long-term phase comprising infusion of a dose of selenite of 0.333-1.1 mg/m² per hour for at least 24 hours.

Thus, one embodiment of the present invention relates to a combinatory dosage regime of an anti-tumour agent and sodium selenite, wherein the internalised daily dose of the sodium selenite is more than 2.0 mg/m², but less than 27.0 mg/m².

To secure the sufficient growth inhibitory effect on tumour cells, another embodiment of the present invention relates to a combinatory dosage regime of an anti-tumour agent and sodium selenite, wherein the internalised daily dose of the selenite is more than 3.0 mg/m², but less than 16.0 mg/m², such as but not limited to an internalised daily dose of more than 4.0 mg/m², but less than 16.0 mg/m², more than 5.0 mg/m², but less than 16.0 mg/m², more than 6.0 mg/m², but less than 16.0 mg/m², more than 7.0 mg/m², but less than 16.0 mg/m², more than 8.0 mg/m², but less than 16.0 mg/m², more than 9.0 mg/m², but less than 16.0 mg/m², more than 10.0 mg/m², but less than 16.0 mg/m².

As an alternative maximum-tolerated dose (MTD), the present inventors also suggest a lesser MTD of 12.8 mg/m2.

Thus, in one embodiment, the present invention relates to a combinatory dosage regime of an anti-tumour agent and sodium selenite, wherein the internalised daily dose of the selenite is more than 3.0 mg/m2, but less than 12.8 mg/m2, such as but not limited to an internalised daily dose of more than 4.0 mg/m2, but less than 12.8 mg/m2, more than 5.0 mg/m2, but less than 12.8 mg/m2, more than 6.0 mg/m2, but less than 12.8 mg/m2, more than 7.0 mg/m2, but less than 12.8 mg/m2, more than 8.0 mg/m2, but less than 12.8 mg/m2, more than 9.0 mg/m2, but less than 12.8 mg/m2, more than 10.0 mg/m2, but less than 12.8 mg/m2.

As shown in the present application, the intravenously administered sodium selenite in terminal cancer patients was well tolerated in these patients at doses up to 10.2 mg/m², therefore defined as an alternative maximum-tolerated dose (MTD). At this dose and below, there were limited incidences of adverse effects. Routine blood sample analyses indicated limited side effects, being transient in nature if observed at all. Notably, no bone marrow toxicity was evident.

Thus, in one embodiment, the present invention relates to a combinatory dosage regime of an anti-tumour agent and sodium selenite, wherein the internalised daily dose of the selenite is more than 3.0 mg/m², but less than 10.2 mg/m², such as but not limited to an internalised daily dose of more than 4.0 mg/m², but less than 10.2 mg/m², more than 5.0 mg/m², but less than 10.2 mg/m², more than 6.0 mg/m², but less than 10.2 mg/m², more than 7.0 mg/m², but less than 10.2 mg/m², more than 8.0 mg/m², but less than 10.2 mg/m², more than 9.0 mg/m², but less than 10.2 mg/m², more than 10.0 mg/m², but less than 10.2 mg/m².

### Escalating dose schedule

In one embodiment, the sodium selenite is administered in escalating dose levels during the long-term phase according to a predefined schedule.

### Cancer

The present invention can be used for preventing progression and/or for treating cancers or tumours in a human subject.

In the present context, cancers or tumours includes, but are not limited to, malignant mesoteliomas, squamous cell carcinomas, adenocarcinomas, thymomas and thymuscarcinomas, mainly stages III and IV but also in appropriate cases stages I and II, adenocarcinomas, melanomas, lymphomas, sarcomas, leukaemia, and different organ tumours like lung, breast, ovarian, head and/or neck, prostate, cervical, endometrial, colorectal, gastric, liver, fallopian tubes, esophagus, small intestine, pancreas, kidney, adrenal, vaginal, vulvar, brain and testicular tumours.

A steady-state plasma concentration of sufficient duration is necessary to achieve efficient chemotherapeutic effects. While addressing this, the inventors of the present invention found out that a human lung-lining cancer cell line is highly sensitive to selenite when exposed to lower concentrations for longer periods of time as described in the long-term phase above.

Thus, in one particular preferred embodiment, the invention relates to the treatment of cancer, wherein the cancer is a lung-lining cancer.

The present invention is also particular useful in late stage or aggressive cancer forms.

One useful embodiment of the present invention relates to the use in preventing progression and/or treating malignant mesotheliomas.

The present inventors also disclose effects on mesothelioma, which is an aggressive cancer affecting the membrane lining of the lungs and abdomen. In this type of cancer where treatment options are limited, the use of the combination dosage of the present invention is an efficient complement to cytostatic regimes.

Thus, in one embodiment, the present invention relates to the treatment of cancer, wherein the cancer is mesothelioma, which is a type of cancer that may develop in the tissues covering the lungs or the abdomen.

In conclusion, selenite may be a highly interesting option to the treatment of tumours and an efficient complement to cytostatic regimes.

### Therapy resistance

Cancer therapeutic resistance occurs as cancers develop resistance to treatments such as chemotherapy, radiotherapy and targeted therapies, through many different mechanisms.

Multidrug resistance, the principal mechanism by which many cancers develop resistance to chemotherapy drugs, is a major factor in the failure of many forms of chemotherapy. It affects patients with a variety of blood cancers and solid tumours, including breast, ovarian, lung, and lower gastrointestinal tract cancers.

Tumours usually consist of mixed populations of malignant cells, some of which are drug-sensitive while others are drug-resistant. Chemotherapy kills drug-sensitive cells but leaves behind a higher proportion of drug-resistant cells. As the tumour begins to grow again, chemotherapy may fail because the remaining tumour cells are now resistant.

Patients with therapy-resistant tumours have lower survival rates than patients with drug-sensitive cells. Thus, this patient group would greatly benefit from the combination of selenite of the disclosed dosage regime of the present invention and optionally a cytostatic agent.

The present invention discloses that several patients who had become resistant to chemotherapy experienced renewed sensitivity to subsequent chemotherapy and even reports a complete remission in a patient previously diagnosed with a therapy-resistant tumour.

The present invention is therefore also useful when a patient has become resistant to the chemotherapeutic agents used in the treatment. The present intervention is especially useful in cancers resistant to chemotherapy. Thus, in one embodiment, the present invention relates to patients or a group of patients having one or more therapy-resistant cancer form.

### Cytostatic agent

Chemotherapy drugs that are most often associated with resistance are paclitaxel, docetaxel, vinorelbine, vincristine, vinblastine, doxorubicin, daunorubicin, epirubicin, etoposide, teniposide, topotecan, dactinomycin, and mitomycin C. These drugs are used to treat a wide variety of cancers ranging from those producing solid tumours to those like lymphoma. These drugs also derive from several different families of chemotherapy drugs, so the phenomenon of drug resistance is not confined to one family of cancer drugs or one type of cancer.

Thus, in one embodiment, the present invention relates to a combination dosage regime comprising a specified amount of sodium selenite and a cytostatic agent selected from the group consisting of paclitaxel, docetaxel, vinorelbine, vincristine, vinblastine, doxorubicin, daunorubicin, epirubicin, etoposide, teniposide, topotecan, dactinomycin, and mitomycin C.

The majority of anti-cancer drugs act as cytotoxic drugs. While chemotherapeutic agents have proven extremely useful in the treatment of cancer, nearly all of them are associated with significant toxic effects because of their potential to kill cancerous as well as healthy cells. The toxicity associated with anti-cancer drugs often forces discontinuation of treatment, which may negatively impact the prognosis of a patient's condition and the clinical outcome and result in compromising the quality of life.

The present invention comprises the steps of combining conventional chemotherapy with the administration of sodium selenite in a dosage regimen according to the present invention.

One or more chemotherapeutic agents may be used according to the criteria well known in the art of cancer chemotherapeutics. The dosage and administrative regimens of the chemotherapeutics are well within the purview of those skilled in the art.

This invention is useful for increasing the anti-tumour activity of anti-cancer agents including fluoropyrimidines, pyrimidine nucleosides, purines, platinum analogues, antroacyclines, podophyllotoxins, camptothecins, hormones and hormone analogues, enzymes, proteins and antibodies, vinca alkaloids, taxanes. The anti-cancer agents of the present invention generally fall into one or more of the following functional categories: antihormones, antifolates, antimicrotubule agents, alkylating agents, antimetabolites, antibiotics, topoisomerase inhibitors and antivirals. While the present method for increasing anti-tumour activity is applicable for any chemotherapeutic agent, some exemplary ones are irinotecan, FU, taxol, cisplatin adriamycin, oxaliplatin, cyclophasphamide, taxotere, and EGF and VGF inhibitors.

Selenite can be used together with alkylating agents, two antimetabolites, one-anti-microtubule agent, and one topoisomerase inhibitor. Thus, one embodiment of the present invention relates to a combination dosage regime comprising a specified amount of sodium selenite and an anti-cancer agent selected from the group consisting of alkylating agents, antimetabolites, anti-microtubule agents, and topoisomerase inhibitors.

In a functional definition, any chemotherapy drug that leads to increased respiration of the cell and an increase of free radicals would be contemplated by the present invention.

In one embodiment, targeted therapy, immune therapy and repair inhibiting drugs, like PARP inhibitors, are also envisaged in the present invention.

In one presently preferred embodiment, the cancer chemotherapeutics comprises carboplatin, gemcitabine and cisplatin.

In addition, the present invention can also be used for increasing the anti-tumour activity of other anti-cancer therapies such as radiation treatment.

The combination regimen of an anti-tumour agent and a dosage regimen of sodium selenite may be used with other anti-cancer therapies such as radiation, surgery and immunotherapy.

### Pre-treatment

In a preferred embodiment, the administration of sodium selenite is initiated at least 7 days before treatment with a cytostatic agent.

### Medical kit

The present disclosure also relates to a medical kit for treating a cancer patient, said kit comprising:
(a) a supply of inorganic selenium compound; and
(b) printed instructions for administering the inorganic selenium compound in the dosage regimen according to the present invention.

One embodiment of the present disclosure relates to a kit comprising:
- one vial containing inorganic selenium at a specific concentration,- a second vial containing inorganic selenium at a specific concentration, wherein the concentration is different from the concentration in the first vial,
- optionally one or more vials that are the same as the second vial, and
- instructions to apply the vials in the kit according to any of claims 1-14.

The present disclosure also relates to a medical kit for treating a cancer patient, said kit comprising:
(a) a supply of inorganic selenium compound;
(b) a supply of a chemotherapeutic agents; and
(c) printed instructions according to the present invention for administering inorganic selenium compound and chemotherapeutic agent to a cancer patient.

### In general

It should be understood that any feature and/or aspect discussed above in connection with the compounds according to the invention applies by analogy to the methods described herein.

The terms "anti-tumour agent", "anti-cancer agents", "anti-cancer drugs", "cytostatic agent", "chemotherapeutic agent" etc. are used interchangeably.

The following examples are provided below to illustrate the present invention. They are intended to be illustrative and are not to be construed as limiting in any way.

### EXAMPLES

### PATIENTS AND METHODS

### Patient Eligibility

Inclusion criteria comprise confirmed malignant diseases with progressing tumour in spite of treatment with established drugs for the respective diagnosis, capacity to cooperate, a good or fairly good performance status (WHO performance status 0-2), and patients over 18 years of age. Any other complicating disease, such as severe heart disease or brain metastases, interfering with the possibility of carrying out the study and any other cancer treatment three weeks prior to the treatment start, exclude the patient. All patients have provided informed consent prior to receiving the treatments.

### Study Design

This phase I trial is an open-label dose-escalation study of i.v. administered sodium selenite. Each cohort consists of three patients who are treated with dosages regimens according to a predefined schedule having the two-step dosage regimen.

### Drug, Dose Escalation Design and Infusion Schedule

GMP grade sodium selenite (Intro-Selen i.v., Pharma Nord ApS, Denmark) was used for the study. The first cohort in this study is given 9.5 mg/m² sodium selenite and if this can be given without dose limiting toxicity (DLT)- the next cohort receives approximately 50% higher dose.

The treatment is given as an upload during 3 hours (1.5 mg/m²) and after that, a continuous dose of 4 mg/m²/24 hours each of the 2 next days.

A PET-CT scan is taken the day after infusion stop and evaluated. When there is not observed any increased SUV (somatic uptake value) in the tumour indicating tumour growth, two more selenite infusions are given during the next 2 weeks.

If the tumour grows according to PET-CT scan after the first infusion, no more selenite is given.

### Toxicity Assessment

Serious toxicity in primarily the brain and the heart has been recorded in patients treated with high doses of selenite.

Symptoms of brain toxicity may be confusion, hallucination and decreased consciousness or even unconsciousness.

Heart toxicity has been registered as hypoxia of the heart and moderately increased troponin T and as symptoms pressure in the breast and anxiousness.

In patients with high doses of selenite, symptoms of tumour necrosis have been observed, essentially tumour swelling a few hours after treatment.

In tumour cell cultures, one important way by which selenite may induce cell death is by apoptosis and necrosis. In a clinical situation, the mechanisms for tumour cell death might be a bit different with more necrosis. When the cells die due to apoptosis, the cells will shrink, whereas when the cells die due to necrosis, the cells will swell before rupturing and dying. This will cause the whole tumour to swell, and in one patient suffering from a tumour behind his eye, the tumour swelled to such an extent that the patient was afraid his eye might fall out.

In another patient with brain metastases near the medulla oblongata, the tumour swelled to such an extent that it pressed against the breathing centre, and the patient stopped breathing for a short time and needed breathing support.

It has also been observed that whole cytokeratin 18 (M65) reflecting necrosis seems to better describe the clinical course than cytokeratin fragments (M30) that mainly describe apoptosis.

Important features with this kind of treatment:
1. Shorten the time during which there might be a risk of tumour growth stimulation from selenite infusion as much as possible.
2. Be aware of the risk of especially brain and heart toxicity from the selenite as well as risks or symptoms of acute tumour necrosis that high selenite doses might imply.

With a few exceptions, all patients visited the clinic before and after infusion, at least once a week during the treatment, before each chemotherapy treatment and at the end of treatment. At these occasions, the medical history is recorded, and a physical examination is performed. Patients undergo CT or PET-CT examination one week before the start, three days after the completion of the selenite treatment and after the final chemotherapy treatment. Routine blood parameters are analysed before, during and after the treatment. The ECG is examined before the start and after both the selenite and chemotherapy treatments.

### Sample collection and selenium analysis

Blood samples are collected about 5 minutes prior and post infusion at all treatments. The plasma fractions are obtained by centrifugation and stored at -70°C until analyses. Selenium (as 82Se) in the plasma samples is quantified by ICP-MS, as described previously.

### Pharmacokinetic analyses

Pharmacokinetic analyses are performed by using the Win-Nonlin program Standard Edition version 1.5 (Pharsight Corporation, Mountain View, CA, USA).

The one compartment infusion model was used for the evaluation of the pharmacokinetics. The reciprocal concentrations were used as weights for the iterative procedure. The output from the curve-fitting program indicates the area under the plasma selenium concentration-time curve (AUC) for a single dose, calculated from the fitted zero-time intercepts and exponential rate constants of a one exponential function giving AUC values from time zero to infinity. AUC values during the entire treatment period (i.e. from day 0 to day 35) were evaluated by applying the trapezoidal rule to estimated time-concentration curves.

### Patients

Patients received at least two doses of selenite according to the two days continuous treatment schedule in Table 1 and were evaluable for toxicity.

### Example 1 - Pharmacokinetics

The pharmacokinetic modelling was successful for data from all patients receiving all or most of the treatment. There was a close correlation between the observed and predicted selenium concentration.

### Example 2 - Clinical assessment

The anti-tumour effect was a secondary endpoint. Tumour size (RECIST) is recorded with PET-CT scan with contrast just before start of treatment, A PET-CT scan is taken immediately after the first selenite treatment and a CT scan after the following selenite treatments. One further CT scan is taken after the chemotherapy. The results from these measurements are presented in waterfall plot.

None of the patients had a response immediately after their respective treatments, but several patients had a stable disease (SD) after selenite treatment and several patients after chemotherapy. In some cases, the tumour disappeared as revealed by CT and PET-CT examinations.

In PET-CT examination with [18F]-2-fluoro-2-deoxy-D-glucose, (FDG) was performed before and at the end of the selenite treatment. In some patients, SUVmax (Standardized Uptake Value for deoxyglucose in the tumour) increased after selenite treatment, indicating tumour-progression.

In some patients, there was no change.

In some patients, there were indications of a decreased uptake, albeit with some decreased uptake in the normal tissues. Some patients with e.g. ethmoidal cancer experienced a clear decrease in SUV, indicating a prominent decrease in the liver metastasis burden.

In some patients with pleuritis, it was possible to address the modulatory effects of the selenite treatment on the response of subsequent chemotherapy *ex vivo* following isolation of the primary tumour cells from the pleura exudate. This was done before and after the selenite treatment. The cells were treated with carboplatin or gemcitabine and their combination *ex vivo.* The tumour cells were more sensitive to the combined carboplatin and gemcitabine treatment after the selenite treatment.

These patients had clinically and radiologically determined tumour progression during the selenite treatment, but regression when later treated with carboplatin and gemcitabine in combination.

### Example 3 - Isolation of tumour cells and chemotherapy drug treatment

Pleura fluid was collected from a patient with pleuritis before receiving the first selenite injections and after the last injection. The fluid was centrifuged at 1100 rpm for 5 min. The cell pellets were washed several times with PBS and transferred to culture medium (Iscove's Modified Dulbecco's Medium, Sigma, Sweden) containing 20% heat inactivated foetal calf serum (FBS), 5% PEST and gentamycin (20 µg/mL) and cultured at 37°C in a humidified atmosphere with 5% CO2.

Since many inflammatory cells were present, the cells were washed with PBS regularly until only adherent cells remained in the culture. When the cells started to proliferate, the FBS concentration was reduced to 10%. For the experimental set up, cells were counted and seeded into a 96-well plate (Sarstedts, Sweden) at density of 7000 cells /well and incubated for 48 h. Cells were washed carefully with PBS and treated with either Carboplatin or Gemcitabine, or in combination for 48 hours. The cell viability was evaluated by XTT (Roche, Germany). All the cell experiments were completed between passages 2-4.

### Example 4 - Selenite toxicity to A549 cells

A549 cells (ATCC) were seeded at a density of 1x105 cells/mL in T-75 flasks (Sarstedts, Sweden) and maintained for 24 hours prior to selenite exposure. Cells were washed once with PBS and exposed to sodium selenite spiked in the medium. Following exposure termination, cells were washed twice with excess of PBS and trypsinized. The detached cells were washed again with ice cold PBS for few times and divided into two aliquots for protein and selenium measurement. For toxicity experiment, these cells were cultured in 96-well plate at a density of 5×104 cells in 100 µL volume. Prior to selenite exposure, cells were washed once with PBS and exposed to different selenite-spiked media for 24 hours. The cell viability was measured by WST-1 (Roche, Germany) kit.

### Example 5 - Measurement of cytokeratin 18

Caspase cleaved cytokeratin 18 (reflecting apoptosis, M30) and total cytokeratin 18 (cleaved and whole, the latter reflecting necrosis, M65) were measured in plasma by using a commercial sandwich quantitative enzyme-linked immunosorbent assay (ELISA); the Peviva M65^{®} ELISA (VLVBio AB, Sundbyberg, Sweden), according to the protocol of the manufacturer. All experiments were performed blindly to clinical correlates. The protein levels of CK18 were expressed as U/L.

### Example 6 - Quality control for selenium measurements

The relative standard deviation was 3.8 % for samples containing 50 ng Se/mL or higher. The accuracy of the analyses was assured by analysing the Se concentration in the certified reference material (CRM) BCR 637 Human Serum in parallel with the study samples. The values found for the CRM (89 ± 1 ng Se/mL) did not deviate significantly from the certified value (80 ± 7 ng Se/mL).

## Claims

1. Sodium selenite for use in the treatment of cancer, wherein the sodium selenite is administered in a dosage regime comprising:
a) a short loading phase comprising infusion of a dose of sodium selenite of 0.125-3 mg/m² per hour for less than 5 hours wherein a total dose of 3-9 mg/m² is administered during a period of 3 hours followed by
b) a long-term phase comprising infusion of a dose of sodium selenite of 0.333-1.1 mg/m² per hour for at least 24 hours,
wherein the infusion dose of sodium selenite per hour in the short loading phase is at least 2 times higher than the infusion dose of sodium selenite per hour in the long-term phase.

2. Sodium selenite for use according to claim 1, wherein the human subject is further treated with a cytostatic agent.

3. Sodium selenite for use according to any of claims 1-2, wherein the human subject is treated with sodium selenite for a time period of at least 3 days before treatment with the cytostatic agent.

4. Sodium selenite for use according to any of claims 1-3, wherein the sodium selenite is GMP grade sodium selenite.

5. Sodium selenite for use according to any of claims 1-4, wherein the cancer is malignant mesothelioma.

6. Sodium selenite for use according to any of claims 1-5, wherein the short loading phase infusion dose of the sodium selenite is selected from the group consisting of an infusion solution administered in a dose of 0.1333 mg/m² per hour, 0.2667 mg/m² per hour, 0.4 mg/m² per hour, 0.5 mg/m² per hour, 0.7667 mg/m² per hour, 1.0 mg/m² per hour, 1.2667 mg/m² per hour, 1.5 mg/m² per hour, 1.7667 mg/m² per hour, and 2.0 mg/m² per hour.

7. Sodium selenite for use according to any of claims 1-6, wherein the long term phase infusion dose of sodium selenite is selected from the group consisting of an infusion solution administered in a dose of 0.3333 mg/m² per hour, 0.4167 mg/m² per hour, 0.5 mg/m² per hour, 0.5833 mg/m² per hour, and 0.6667 mg/m² per hour.

8. Sodium selenite for use according to any of claims 1-7, wherein the long-term phase consists of an infusion solution administered in a total dose of 2-32 mg/m² during a period of 48 hours.

9. Sodium selenite for use according to any of claims 1-8, wherein the short loading phase consists of an infusion solution administered in a total dose of 0.4-6 mg/m² during a period of 3 hours.

10. Sodium selenite for use according to any of the claims 1-9, wherein the resulting plasma concentration of the sodium selenite is between 8-30 µM.

11. Sodium selenite for use according to any of the claims 1-10, wherein treatment regimen is repeated one, two, or three times.

12. Sodium selenite for use according to any of the claims 1-11, wherein the administration pattern is followed by an evaluation step, the evaluation step preferably consisting of a PET-CT scanning.

13. Sodium selenite for use according to any of the claims 1-12, wherein the treatment with sodium selenite is stopped if the evaluation step discloses disease progression.

## Patentansprüche

1. Natriumselenit zur Verwendung bei der Behandlung von Krebs, wobei das Natriumselenit in einem Dosierungsschema verabreicht wird, das Folgendes umfasst:
a) eine kurze Aufladephase, umfassend die Infusion einer Dosis Natriumselenit von 0,125-3 mg/m² pro Stunde über einen Zeitraum von weniger als 5 Stunden, wobei eine Gesamtdosis von 3-9 mg/m² über einen Zeitraum von 3 Stunden verabreicht wird, gefolgt von
b) einer Langzeitphase, umfassend die Infusion einer Dosis Natriumselenit von 0,333-1,1 mg/m² pro Stunde über einen Zeitraum von mindestens 24 Stunden,
wobei die Infusionsdosis an Natriumselenit pro Stunde in der kurzen Aufladephase mindestens doppelt so hoch ist wie die Infusionsdosis an Natriumselenit pro Stunde in der Langzeitphase.

2. Natriumselenit zur Verwendung nach Anspruch 1, wobei das menschliche Subjekt zusätzlich mit einem Zytostatikum behandelt wird.

3. Natriumselenit zur Verwendung nach einem der Ansprüche 1-2, wobei das menschliche Subjekt vor der Behandlung mit dem Zytostatikum über einen Zeitraum von mindestens drei Tagen mit Natriumselenit behandelt wird.

4. Natriumselenit zur Verwendung nach einem der Ansprüche 1-3, wobei es sich bei dem Natriumselenit um Natriumselenit in GMP-Qualität handelt.

5. Natriumselenit zur Verwendung nach einem der Ansprüche 1-4, wobei der Krebs ein malignes Mesotheliom ist.

6. Natriumselenit zur Verwendung nach einem der Ansprüche 1-5, wobei die Infusionsdosis des Natriumselenits für die kurze Aufladephase aus der Gruppe ausgewählt wird, die aus einer Infusionslösung besteht, die in einer Dosis von 0,1333 mg/m² pro Stunde, 0,2667 mg/m² pro Stunde, 0,4 mg/m² pro Stunde, 0,5 mg/m² pro Stunde, 0,7667 mg/m² pro Stunde, 1,0 mg/m² pro Stunde, 1,2667 mg/m² pro Stunde, 1,5 mg/m² pro Stunde, 1,7667 mg/m² pro Stunde und 2,0 mg/m² pro Stunde verabreicht wird.

7. Natriumselenit zur Verwendung nach einem der Ansprüche 1-6, wobei die Infusionsdosis von Natriumselenit für die Langzeitphase aus der Gruppe ausgewählt wird, die aus einer Infusionslösung besteht, die in einer Dosis von 0,3333 mg/m² pro Stunde, 0,4167 mg/m² pro Stunde, 0,5 mg/m² pro Stunde, 0,5833 mg/m² pro Stunde und 0,6667 mg/m² pro Stunde verabreicht wird.

8. Natriumselenit zur Verwendung nach einem der Ansprüche 1-7, wobei die Langzeitphase aus einer Infusionslösung besteht, die über einen Zeitraum von 48 Stunden in einer Gesamtdosis von 2-32 mg/m² verabreicht wird.

9. Natriumselenit zur Verwendung nach einem der Ansprüche 1-8, wobei die kurze Aufladephase aus einer Infusionslösung besteht, die über einen Zeitraum von 3 Stunden in einer Gesamtdosis von 0,4-6 mg/m² verabreicht wird.

10. Natriumselenit zur Verwendung nach einem der Ansprüche 1-9, wobei die resultierende Plasmakonzentration des Natriumselenits zwischen 8-30 µM liegt.

11. Natriumselenit zur Verwendung nach einem der Ansprüche 1-10, wobei der Behandlungsplan ein-, zwei- oder dreimal wiederholt wird.

12. Natriumselenit zur Verwendung nach einem der Ansprüche 1-11, wobei auf das Verabreichungsmuster ein Auswertungsschritt folgt, wobei der Auswertungsschritt vorzugsweise aus einer PET-CT-Untersuchung besteht.

13. Natriumselenit zur Verwendung nach einem der Ansprüche 1-12, wobei die Behandlung mit Natriumselenit abgebrochen wird, wenn der Auswertungsschritt ein Fortschreiten der Erkrankung offenbart.

## Revendications

1. Sélénite de sodium pour une utilisation dans le traitement du cancer, dans lequel le sélénite de sodium est administré selon un régime posologique comprenant :
a) une courte phase de mise en charge comprenant la perfusion d'une dose de sélénite de sodium de 0,125 à 3 mg/m² par heure pendant moins de 5 heures dans laquelle une dose totale de 3 à 9 mg/m² est administrée pendant une période de 3 heures suivie de
b) une phase de longue durée comprenant la perfusion d'une dose de sélénite de sodium de 0,333 à 1,1 mg/m² par heure pendant au moins 24 heures,
dans lequel la dose de perfusion de sélénite de sodium par heure dans la courte phase de mise en charge est au moins 2 fois supérieure à la dose de perfusion de sélénite de sodium par heure dans la phase de longue durée.

2. Sélénite de sodium pour une utilisation selon la revendication 1, dans lequel le sujet humain est en outre traité avec un agent cytostatique.

3. Sélénite de sodium pour une utilisation selon l'une quelconque des revendications 1 à 2, dans lequel le sujet humain est traité avec du sélénite de sodium pendant une période d'au moins 3 jours avant le traitement avec l'agent cytostatique.

4. Sélénite de sodium pour une utilisation selon l'une quelconque des revendications 1 à 3, dans lequel le sélénite de sodium est du sélénite de sodium de qualité BPF.

5. Sélénite de sodium pour une utilisation selon l'une quelconque des revendications 1 à 4, dans lequel le cancer est un mésothéliome malin.

6. Sélénite de sodium pour une utilisation selon l'une quelconque des revendications 1 à 5, dans lequel la dose de perfusion à courte phase de mise en charge du sélénite de sodium est sélectionnée dans le groupe consistant en une solution de perfusion administrée à une dose de 0,1333 mg/m² par heure, 0,2667 mg/m² par heure, 0,4 mg/m² par heure, 0,5 mg/m² par heure, 0,7667 mg/m² par heure, 1,0 mg/m² par heure, 1,2667 mg/m² par heure, 1,5 mg/m² par heure, 1,7667 mg/m² par heure et 2,0 mg/m² par heure.

7. Sélénite de sodium pour une utilisation selon l'une quelconque des revendications 1 à 6, dans lequel la dose de perfusion à phase de longue durée de sélénite de sodium est sélectionnée dans le groupe consistant en une solution de perfusion administrée à une dose de 0,3333 mg/m² par heure, 0,4167 mg/m² par heure, 0,5 mg/m² par heure, 0,5833 mg/m² par heure et 0,6667 mg/m² par heure.

8. Sélénite de sodium pour une utilisation selon l'une quelconque des revendications 1 à 7, dans lequel la phase de longue durée consiste en une solution de perfusion administrée à une dose totale de 2 à 32 mg/m² pendant une période de 48 heures.

9. Sélénite de sodium pour une utilisation selon l'une quelconque des revendications 1 à 8, dans lequel la courte phase de mise en charge consiste en une solution de perfusion administrée à une dose totale de 0,4 à 6 mg/m² pendant une période de 3 heures.

10. Sélénite de sodium pour une utilisation selon l'une quelconque des revendications 1 à 9, dans lequel la concentration plasmatique résultante du sélénite de sodium est comprise entre 8 et 30 µM.

11. Sélénite de sodium pour une utilisation selon l'une quelconque des revendications 1 à 10, dans lequel le régime de traitement est répété une, deux ou trois fois.

12. Sélénite de sodium pour une utilisation selon l'une quelconque des revendications 1 à 11, dans lequel le schéma d'administration est suivi d'une étape d'évaluation, l'étape d'évaluation consistant préférablement en un balayage PET-CT.

13. Sélénite de sodium pour une utilisation selon l'une quelconque des revendications 1 à 12, dans lequel le traitement avec du sélénite de sodium est arrêté si l'étape d'évaluation révèle une progression de la maladie.
